# EUROPEAN PATENT SPECIFICATION

(11) **EP 2 945 494 B1**
(45) Date of publication and mention of the grant of the patent: **08.03.2017**
(21) Application number: 14700426.1
(22) Date of filing: 14.01.2014
(51) Int. Cl.: A61P 1/12, A61K 35/74, A23L 33/00, A23L 33/135

(54) **BABY FOOD COMPOSITION COMPRISING VIABLE PROPIONIC ACID-PRODUCING BACTERIA AND VIABLE LACTIC ACID-PRODUCING BACTERIA**
SÄUGLINGSNAHRUNGSMITTELZUSAMMENSETZUNG, ENTHALTEND LEBENSFÄHIGE PROPIONSÄURE-PRODUZIERENDE BAKTERIEN SOWIE LEBENSFÄHIGE MILCHSÄURE-PRODUZIERENDE BAKTERIEN
COMPOSITION D'ALIMENT POUR BÉBÉS COMPRENANT DES BACTÉRIES VIABLES PRODUISANT DE L'ACIDE PROPIONIQUE ET DES BACTERIES VIABLES PRODUISANT DE L'ACIDE LACTIQUE

(30) Priority: 21.01.2013 EP 13000283
(43) Date of publication of application: 25.11.2015
(73) Proprietor: ETH Zürich, 8092 Zürich (CH); Universität Zürich, 8006 Zürich (CH)
(72) Inventor: CHASSARD, Christophe, 63370 Lempdes (CH); LACROIX, Christophe, CH-8802 Kilchberg (CH); BRAEGGER, Christian, CH-8032 Zürich (CH)
(74) Representative: Grimm, Siegfried
(86) International application number: PCT/CH2014/000006
(87) International publication number: WO 2014/110685

(56) References cited:
- EP-A1- 1 308 506
- EP-A1- 1 374 878
- WO-A1-2012/059502
- US-A1- 2004 062 758
- US-A1- 2005 180 963
- US-A1- 2007 258 953
- US-A1- 2010 166 721
- US-A1- 2012 171 166

## Description

The present invention relates to compositions, particularly baby food compositions, comprising viable lactic acid-producing bacteria from one or more live bacteria strains, and viable lactate utilising, propionic acid-producing bacteria from one or more live bacteria strains, its manufacture and use in the treatment of digestive diseases, particularly for treatment of infantile colics (IC).

It is well known that human milk from healthy and well-nourished mothers provides adequate nutrition for infants during the first few months of life and also significantly reduces the risk of acute and chronic diseases during this critical development period. For infant nutritional products, the chemical composition of human milk served as a guide for the formulation of infant formula. In recent years, selected physiological outcomes have gained acceptance in documenting the functionality of novel ingredients, including some chemical entities not found in human milk.

Callaghan et al (Infant Formulae, in: Encyclopedia of Dairy Sciences (2nd Ed), Editor: John W. Fuquay, Elsevier Ltd, pp 135-145, incorporated by reference) summarizes regulations governing the manufacture, composition, and labeling of infant nutritional products. These regulations ensure safety and efficiency of the products but also provide limitations for new nutritional products.

A number of infant nutritional products, comprising probiotics, are commercially available. These products involve selected lactate-producers (either Bifidobacterium or Lactobacillus): Nestle Good start® supplemented with Bifidobacterium lactis; Lactogen 3® supplemented with Lactobacillus reuteri; Guigoz croissance 3® supplemented with Lactobacillus reuteri. Further, a number of documents relate to nutritional products comprising bacteria and having a therapeutic use.

WO2004/085628 describes lactic acid utilizing bacteria and their therapeutic use, particularly for treatment of inflammatory diseases.

WO011/020780 describes nutritional compositions comprising lactococcus strains and their therapeutic use, particularly for the treatment of allergy symptoms. US2010/0166721 describes probiotic compositions and in very general terms its use as a food supplement for normalization of the gastointestinal flora.

EP1374878 describes methods and compositions for preventing or alleviating symptoms of malabsorption from the GI tract.

US2004/0062758 describes a combination of probiotics and its use for stimulating the immune system and for general health improvement.

WO2012/059502 describes a powdered cereal based composition comprising probiotic micro-organisms and its use in strengthening the immune system or treatment of inflammatory disorders. The document is specifically directed to non-replicating micro-organisms. US2012/0171166 describes symbiotic combination of specific oligosaccharides to promote growth of beneficial microbiota and its use for treating GI disorders. The document is specifically directed to butyrate producing bacteria.

US2007/0258953 describes probiotic compositions comprising viable, novel lactic acid utilizing bacteria; as well as their use as a medicament.

US2005/0180963 describes probiotic compositions comprising viable, novel proprionibacteria; as well as their use in the treatment of GI diseases.

None of the above documents address the treatment of digestive diseases or disorders selected from the group consisting of colics, intestinal discomfort, intestinal pain, visceral sensitivity and intestinal cramp, particularly the treatment of infantile colics. Even more, it is believed there is no established treatment of infantile colics known today. In consequence, there is an unmet clinical need.

Thus, it is an object of the present invention to address this clinical need and to overcome at least some of these drawbacks of the state of the art. In particular, it is an aim of the present invention to provide food / pharmaceutical compositions for treatment of digestive diseases, such as IC as well as methods for treatment of the above disorders.

These objectives are achieved by the composition as defined in claim 1. Further aspects of the invention are disclosed in the specification and independent claims, preferred embodiments are disclosed in the specification and the dependent claims.

The present invention will be described in more detail below. It is understood that the various embodiments, preferences and ranges as provided / disclosed in this specification may be combined at will. Further, depending of the specific embodiment, selected definitions, embodiments or ranges may not apply.

Unless otherwise stated, the following **definitions** shall apply in this specification:
As used herein, the term "a," "an,", "the" and similar terms used in the context of the present invention (especially in the context of the claims) are to be construed to cover both the singular and plural unless otherwise indicated herein or clearly contradicted by the context.

As used herein, the terms "including", "containing" and "comprising" are used herein in their open, non-limiting sense.

The term "treatment" shall also include the delay of progression as well as the prevention (prophylaxis).

The terms "digestive diseases" and "digestive disorders" are well known and describe diseases/disorders that pertain to the gastrointestinal tract, particularly the gut. Particularly included are infantile colics ("IC"). Diagnostic criteria for IC must include all of the following in infants from birth to 4 months of age: 1. Paroxysms of irritability, fussing or crying that starts and stops without obvious cause 2. Episodes lasting 3 or more hours/day and occurring at least 3 days/wk for at least 1 week 3. No failure to thrive. IC is also known as baby colic, ICD-10: R10.4.

The term "propionic acid-producing bacteria" refers to a group of bacteria that produce propionate as main product from the carbon metabolism.

The term "propionic acid bacteria", synonymous for "Propionibacteria" is well known and established in the field; they are named for their unique metabolism leading to propionic acid as a major end product of metabolism. Such bacteria may ferment a large number of substrates, including lactate. The major end products of propionic fermentation are propionic, acetic, and succinic acids and CO2. Sugar substrates (glucose mainly) are first oxidized to pyruvate via glycolysis or via the pentose phosphate pathway, generating ATP and reduced coenzymes. Pyruvate is further catabolized via two main pathways, producing either propionate or acetate and CO2. Lactate can be metabolized to propionate by the acrylate pathway where water is removed from lactate to form acrylate with subsequent reduction to propionate (Microbial Physiology. Albert G. Moat, John W. Foster and Michael P. Spector, Wiley-Liss, Inc.
Propionic acid bacteria have a generally recognized as safe status (GRAS) in the United States and a qualified presumption of safety (QPS) status in Europe.
Propionic acid bacteria are classified in the class of Actinobacteria with other Gram-positive bacteria with a GC content higher than 50%.

The terms "Lactate-utilizing, propionic acid-producing bacteria" and "lactic acid producing bacteria" are defined below.

The term "probiotics" is well known and established in the field and particularly relates to microbial cell preparations or components of microbial cells that have a beneficial effect on the health and well-being of humans. Accordingly, the term probiotics encompasses the above defined bacteria.

The term "prebiotic" is well known and established in the field and particularly relates to a non-digestible food ingredient that beneficially affects humans by selectively stimulating the growth and/or activity of one or a limited number of bacteria in the colon, and thus improves human health.

In more general terms, in a first aspect, the invention relates to new compositions comprising as a first component viable lactate-utilizing, propionic acid-producing bacteria. These inventive compositions may be adapted to food compositions, particularly baby food compositions, and / or pharmaceutical compositions. This aspect of the invention shall be explained in further detail below:

Food composition: The inventive compositions may be in the form of any food composition. Preferably, the compositions are adapted to infant, particularly baby, nutrition. Accordingly, the food composition may be in the form of an infant starter formula, a follow-on formula, a baby food formula, an infant cereal formula or a growing-up milk. Such food compositions are known and described e.g. in Callaghan et al (Infant Formulae, in: Encyclopedia of Dairy Sciences (2nd Ed), Editor: John W. Fuquay, Elsevier Ltd, pp 135-145, incorporated by reference)). Typically, such food compositions comprise additives and growth enhancing elements as described below. The term food composition also encompasses an adult nutritional composition or an adult milk-protein based drink for individuals in need of therapy. Preferably, said food composition is a starter infant formula, such as a baby milk or baby milk powder.

Pharmaceutical compositions: The inventive compositions may be in the form of any pharmaceutical formulation, such as solid, semi-solid or liquid formulation. Further, the above food compositions may also be used as a pharmaceutical composition. Pharmaceutical compositions comprise, next to the composition to be administered, also instructions for the administration ("package insert").

Viable (living) bacteria: The use of viable (living) bacteria is believed to be a key feature of the present invention, distinguishing it from the prior art and allowing the uses described herein. According to the invention, the bacteria are viable (living), i.e. they are metabolically active and / or are able to colonize the gut of a mammalian, particularly a human. The term includes both (i) bacteria able to divide and form a colony and (ii) bacteria which are non-replicating.

Bacteria of group (i) are able to divide and form a colony on a nutrient medium appropriate for the growth of the bacteria, or to increase turbidity of liquid growth medium after inoculation with different concentrations of bacterial preparations and incubation under appropriate conditions (aerobic and/or anaerobic atmosphere for at least 24 h). Such classical plating methods are known and described e.g. in Jay et al (Modern Food Microbiology. 7th Edition, Springer, 2005). Bacteria of group (ii) includes live but non-replicating bacteria that can be enumerated with fluorescent stains targeting bacterial membrane potentials or enzymatic activities enabled the differentiation between viable, metabolic active, damaged, dormant, viable but not cultivable, and dead bacterial cells. Molecular tools like fluorescence in situ hybridization (FISH) and flow cytometry were successfully applied to estimate viable cells in probiotic products.

In a preferred embodiment, the invention relates to compositions comprising bacteria of group (i).

In an alternative embodiment, the invention relates to compositions additionally comprising bacteria of group (ii).

In an alternative embodiment, the invention relates to compositions only comprising bacteria of group (ii).

The amount of such bacteria may vary over a broad range and an effective amount may be determined by the skilled person in routine experiments. Amounts effective for this purpose will depend on a number of factors known to those of skill in the art such as the weight and general health state of the human to be treated, and on the effect of the food matrix. Typically, the effective amount in the compositions of the present invention is in the range of 10² to 10¹² cfu/g, more preferably 10⁴ to 10⁹ cfu/g, most preferably 10⁷ to 10⁹ cfu/g composition or per mL of composition.

### Lactate-utilizing, propionic acid-producing bacteria:

According to the present invention, the propionic acid-producing bacteria are selected from the group of "Lactate utilizing propionic acid-producing bacteria". Gut bacteria utilising lactate to produce propionate though the acrylate pathways include Propionibacteria and relatives, Veillonella and relatives, Selomonas and relatives, Megasphaera and relatives and any other lactate utilizing propionate producing bacteria isolated from the infant intestinal ecosystem. The acrylate pathway is known an described in literature, e.g. Hosseini et al. (Nutr. Rev. 9:245-258, 2011), particularly fig. 2, which is incorporated by reference in its entirety.

In a further preferred embodiment, the propionic acid-producing bacteria are selected from the currently known 13 species.

In a further preferred embodiment, the propionic acid-producing bacteria are selected from the group of P. freudenreichii, P. acidipropionici, P. jensenii, P. thoenii, P. cyclohexanicum, and P. microaerophilum.

Accordingly, the invention provides for a composition, particularly a food composition or a pharmaceutical composition, comprising (i) viable lactate utilising, propionic acid-producing bacteria from one or more live bacteria strains, particularly from strains as identified herein; (ii) optionally a source of proteins, (iii) optionally a source of carbohydrates, (iv) optionally a source of lipids; (v) optionally a source of vitamins and minerals; (vi) optionally additives; (vii) optionally water.

The composition according to the present invention typically contains a protein source. Suitable are amounts of not more than 2.0 g/100 kcal, preferably 1.8 to 2.0 g/100kcal. The type of protein is not believed to be critical to the present invention provided that the minimum requirements for essential amino acid content are met and satisfactory growth is ensured although it is preferred that over 50% by weight of the protein source is whey. Thus, protein sources based on whey, casein and mixtures thereof may be used as well as protein sources based on soy. As far as whey proteins are concerned, the protein source may be based on acid whey or sweet whey or mixtures thereof and may include alpha- lactalbumin and beta-lactoglobulin in whatever proportions are desired.

The composition according to the present invention typically contains a carbohydrate source. Any carbohydrate source conventionally found in infant formulae such as lactose, saccharose, maltodextrin, starch and mixtures thereof may be used although the preferred source of carbohydrates is lactose. Preferably the carbohydrate sources contribute between 35 and 65% of the total energy of the composition.

The composition according to the present invention typically contains a source of lipids. The lipid source may be any lipid or fat which is suitable for use in infant formulas. Preferred fat sources include palm olein, high oleic sunflower oil and high oleic safflower oil. The essential fatty acids linoleic and [alpha]-linolenic acid may also be added as may small amounts of oils containing high quantities of preformed arachidonic acid and docosahexaenoic acid such as fish oils or microbial oils. In total, the fat content is preferably such as to contribute between 30 to 55% of the total energy of the formula. The fat source preferably has a ratio of n-6 to n-3 fatty acids of about 5:1 to about 15:1; for example about 8:1 to about 10:1.

The composition according to the invention typically contains all vitamins and minerals understood to be essential in the daily diet and in nutritionally significant amounts. Minimum requirements have been established for certain vitamins and minerals. Examples of minerals, vitamins and other nutrients optionally present in the infant formula include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chloride, potassium, sodium, selenium, chromium, molybdenum, taurine, and L-carnitine. Minerals are usually added in salt form. The presence and amounts of specific minerals and other vitamins will vary depending on the intended infant population.

The composition according to the invention is dry. This ensures a stable composition comprising living bacteria as defined herein. However, low water content, typically below 5%, may be acceptable and is thus comprised within the present invention.

The composition according to the invention may further contain other components which may have a beneficial effect such as fibers, lactoferrin, nucleotides, nucleosides, and the like.

The composition according to the invention may further contain emulsifiers and stabilizers such as soy lecithin, citric acid esters of mono- and di-glycerides, and the like. This is especially the case if the composition is provided in liquid form.

The composition according to the invention may further contain stabilizers (or "stabilizing agents"). This term refers to compounds or materials that are added to the composition to increase the viscosity of the wet formulation or to form a hydrogel. Examples of a suitable stabilizer agent include but are not limited to polysaccharides, such as, cellulose acetate phthalate (CAP), carboxy-methyl-cellulose, pectin, sodium alginate, salts of alginic acid, hydroxyl propyl methyl cellulose (HPMC), methyl cellulose, carrageenan, guar gum, gum acacia, xanthan gum, locust bean gum, chitosan and chitosan derivatives, collagen, polyglycolic acid, starches and modified starches, cyclodextrins and oligosaccharides (inulin, maltodextrins, raffinose, dextrans, etc.) and combinations thereof.

The composition according to the invention may further contain protecting agents (or "protective agents" or "protectants"). This term refers to compounds or materials that are added to ensure or increase the stability of the viable bacteria during the drying process and afterwards, or for long-term storage stability of the dry powder product. Suitable protectants are generally readily soluble in a solution and do not thicken or polymerize upon contact with water. Suitable protectants are described below and include, but are not limited to, proteins such as human and bovine serum albumin, whey protein, soy protein, caseinate, gelatin, immunoglobulins, carbohydrates including monosaccharides (galactose, D-mannose, sorbose, etc.), disaccharides (lactose, trehalose, sucrose, etc.), an amino acid such as monosodium glutamate, lysine, glycine, alanine, arginine or histidine, as well as hydrophobic amino acids (tryptophan, tyrosine, leucine, phenylalanine, etc.); a methylamine such as betaine; an excipient salt such as magnesium sulfate; a polyol such as trihydric or higher sugar alcohols (e.g., glycerin, erythritol, glycerol, arabitol, xylitol, sorbitol, and mannitol); propylene glycol; polyethylene glycol; pluronics; surfactants, and combinations thereof.

The composition according to the invention may further contain prebiotic additives. This term refers to compounds / materials used to stimulate the growth of specific gut microbes, such as the viable bacteria as defined herein. Suitable prebiotics are known to the skilled person and are non-digestible food ingredients that beneficially affect the host by selectively stimulating the growth and / or activity of one or a limited number of bacteria in the colon. Such ingredients are non-digestible in the sense that they are not broken down and absorbed in the stomach or small intestine and thus pass intact to the colon where they are selectively fermented by the beneficial bacteria. Examples of suitable prebiotics include certain oligosaccharides, such as fructooligosaccharides (FOS), acacia gum and galactooligosaccharides (GOS). A combination of prebiotics may be used such as short chain GOS with long chain FOS such as the product sold under the trade mark Immunofortis® (Arslanoglu S et al. J Nutr 2008; 138: 1091-5).

In a preferred embodiment, the invention provides a composition comprising a combination of viable bacteria as defined herein and prebiotics as defined herein. A combination of both components significantly improves the therapeutic and / or prophylactic effects as described herein (synergism).

The composition of the present invention further comprises viable lactic acid-producing bacteria.

Preferably, the lactic acid producing bacteria are selected from the group consisting of Lactic Acid Bacteria and Bifidobacteria. Preferably, lactic acid producing bacteria are selected from the strains consisting of Bifidobacteria, Lactobacilli, Lactococci, Steptococci, Enterococci, Leuconostoc, Weissella. It was found that the combination of lactic acid-producing bacteria and lactate-utilizing bacteria are particularly beneficial for food compositions as defined herein. Again, these lactic acid-producing bacteria are viable as defined herein.

The inventive composition may be provided as liquid composition ready to be administered (i.e. a "baby formula", "baby milk") or as dried composition (i.e. a "baby milk powder", "supplement") to be reconstituted with water prior use. For preservation of viability during storage and until consumption of the probiotic-containing product the probiotic can only be supplied in a dried form with the formula (either liquid or powdered).
In the case of a liquid composition, the viable bacteria are supplied separate from the product in a dried form (a "supplement") which can be added before use. In this embodiment, the invention provides a kit of parts, the first part being is solid dosage form comprising the living bacteria and the second part being a liquid product (such as a baby milk) free of living bacteria.
In the case of a dry composition (such as a milk powder), it is preferred that the composition of the dried infant formula containing the viable bacteria has a water activity below 0.2, preferably below 0.15 to further increase shelf stability. Low water activity reduces the rate of degradation of powders and blocks the growth and activity of microbes. Water activity values below 0.2 which correspond to water content of ca. 5%, preferably lower in the range from 2.5 to 3.5%, or lower are typically used to block lactose crystallization, and considerably decrease the rate of chemical and enzymatic reactions in milk powders.

In a **second aspect,** the invention relates to the use of the inventive composition as pharmaceuticals, particularly for treatment of digestive diseases. The compositions described herein provide a useful bacterial consortium able to promote or re-establish a healthy gut colonization in humans, particularly infants. This aspect of the invention shall be explained in further detail below.

The compositions of the invention have therapeutic and/or preventive effects, and may be used especially for the treatment of digestive diseases in infants or patients in need thereof, or for reducing the risk of digestive diseases in infants or patients in need thereof, or for reducing the severity of digestive diseases in infants or patients in need thereof.

Accordingly, the invention also provides for a composition as described herein as pharmaceutical.

The invention further provides for
▪ the use of a composition as described herein for the manufacture of a medicament for the treatment of digestive diseases or disorders;
▪ the therapeutic use of a composition as described herein;
▪ a method of treatment of digestive disease or disorder comprising the step of administering an effective amount of a composition as described herein to a subject in need thereof;
▪ a composition as described herein for the treatment of digestive diseases or disorders;
▪ a composition as described herein for use in the treatment of digestive diseases or disorders.

The inventive compositions comprise viable bacteria as defined herein in an amount sufficient to at least partially promote a health benefit. An amount to accomplish this is defined as a "therapeutically effective dose". Amounts effective for this purpose will depend on a number of factors known to those of skill in the art such as the weight and general health status of the human to be treated, and on the effect of the food matrix. In prophylactic applications, the inventive compositions are administered to a consumer susceptible to or otherwise at risk of a disorder in an amount that is sufficient to at least partially reduce the risk of developing a disorder as defined herein. Such an amount is defined as a "prophylactic effective dose". Amounts effective for this purpose will depend on a number of factors known to those of skill in the art such as the weight and general health status of the human to be treated, and on the effect of the food matrix.

Without being bound to theory, it is believed that the scientific rational behind the present invention may be summarized as follows:
(1) The infant gut microbiota contains a large and highly diverse population of lactate producing bacteria (mainly Bifidobacteria, Lactobacilli, Streptococci and Staphylococci). Many other species shaping the lactate utilizing community (LUB) also colonize the intestine of infants at early stage, comprising sulfate reducing bacteria (SRB) and non SRB (=LUB). In infants, the LUB population is mainly dominated by *E. hallii* and *Veillonella.* Therefore, an intense competition for lactate occurs in the GIT tract involving *E*. *hallii, Veillonella* and SRB. SRB and *E. halli* dominate the infant intestine microbiota during the first 3 month of life, together with *Veillonella,* which population will further increases at 4 month.
(2) High LUB populations are found and stratified by screaming. LUB are promoted in these infants and there is likely a huge competition for lactate. Screaming could be a consequence of this bacterial competition in the GIT by promoting the growth of certain microbes or production of deleterious bacterial metabolites.
(3) Lactate utilizing propionate producing bacteria could therefore dominate the community at an early stage impacting the global equilibrium of this bacterial community. The objective would be to decrease SRB and *E*. *hallii* populations or metabolites produced. Indeed, SRB produces H2S while *E. hallii* produces a lot of H2 which are both associated to pain and intestinal discomfort in humans.
(4) Lactate utilizing propionate producing bacteria can convert intestinal lactate into propionate and then remove it from the gut ecosystem. This approach prevents any lactate accumulation but also impact growth and metabolism of other lactate utilizing bacteria which produce negative compounds potentially involved in infant screaming and colics.

The present inventors now realized that lactate utilizing propionate producing bacteria could reduce H2S and H2 production in colicky babies by creating a healthier and balance trophic chain in the first month of life. In a preferred embodiment, the digestive disorder therefore is infantile colic (also termed "IC" or "infant colic" or "baby colic").

In a further preferred embodiment, the digestive disease or disorder is selected from the group consisting of colics, intestinal discomfort, intestinal pain, visceral sensitivity and intestinal cramp.

In a further preferred embodiment, the digestive disease is colitis.
In a further embodiment, the inventive compositions are used to prevent lactate accumulation in a patient's (particularly infant's) gut. It is believed that such malfunction may otherwise lead to acidosis and/or colics.

In a further embodiment, the inventive compositions are used to decrease H2S production in a patient's (particularly infant's) gut. It is believed that such use prevents colics and/or colitis.

In a further embodiment, the inventive compositions are used to mimic the trophic chain observed in breast milk. It is believed that this provides optimum nutrition for newborns.

In a further embodiment, the inventive compositions are used to control, or to normalize intestinal colonization and gut ecology. It is believed that this improves gut health.

As outlined above, a particular relevant group of patients are newborn babies. It was found that babies born by caesarean are a particularly important group of patients to be treated. However, the invention is not limited to this specific group of patients, but may be useful for the treatment of (i) newborn babies (typically less than 1 month), (ii) infants (typically less than 12 months) and (iii) young children (typically less than 36 months).

In a **third aspect,** the invention relates to a process for manufacturing a composition as described herein. This aspect of the invention shall be explained in further detail below:
The starting materials, including viable (living) bacteria strains, are known or obtainable according to known methods. The manufacturing of the inventive compositions depend largely on the final product type:
   dry product, ie. a solid form (such as a powder) or wet product, i.e. a liquid (such as a drink) or semi-liquid (such as a mash).

Generally, all methods known for manufacturing food products comprising viable (living) bacteria may be employed. Typically, the food product is produced and a formulation of living bacteria is added.

Generally, the viable bacteria may be cultured according to any suitable method and prepared for addition to the inventive composition by freeze-drying or spray-drying, for example. Details may be found in Lacroix, et al (Microbial production of food ingredients, enzymes and nutraceuticals, McNeil, Giavasis, Harvey Eds. Woodhead Publishing Ltd, Cambridge, (2012)), which is incorporated by reference. Alternatively, bacterial preparations can be obtained from specialist already prepared in a suitable form for addition to the inventive compositions.

In a preferred embodiment, the inventive composition is a milk powder. Milk powder production is a well established technology and described e.g. in Walstra (Dairy science and technology. 2nd ed., Boca Raton : Taylor & Francis, 782 pp. (2006)) and Schuck (Milk Powder: Types and Manufacture. In Encyclopedia of Dairy Sciences (Second Edition), Academic Press, Pages 108-116 (2011)), both incorporated by reference.

For example, an inventive composition may be prepared by blending together protein source, carbohydrate source, and fat source in appropriate proportions. If used, the emulsifiers may be included in the blend. Vitamins and minerals may be added at this point but are usually added later to avoid thermal degradation. Any lipophilic vitamins, emulsifiers and the like may be dissolved into the fat source prior to blending. Water, preferably water which has been subjected to reverse osmosis, may then be mixed in to form a liquid mixture.
The liquid mixture may then be thermally treated to reduce bacterial loads. For example, the liquid mixture may be rapidly heated to a temperature in the range of about 80°C to about 110°C for about 5 seconds to about 5 minutes. This may be carried out by steam injection or by heat exchanger; for example a plate heat exchanger. The liquid mixture may then be cooled to about 60[deg.]C to about 85°C; for example by flash cooling. The liquid mixture may then be homogenised; for example in two stages at about 7 MPa to about 40 MPa in the first stage and about 2 MPa to about 14 MPa in the second stage. The homogenised mixture may then be further cooled to add any heat sensitive components; such as vitamins and minerals. The pH and solids content of the homogenised mixture is conveniently standardised at this point. The homogenised mixture is transferred to a suitable drying apparatus such as a spray drier or freeze drier and converted to powder. The powder should have a moisture content of less than about 5% by weight.

To further illustrate the invention, the following **examples** are provided. These examples are provided with no intend to limit the scope of the invention.

An age-tailored set of nutritional compositions for infant or your children according to the present invention is given below.

| Protein (g/100 kcal) | 2.5 | 2.5 | 2 | 2.5 | 2 |
|---|---|---|---|---|---|
| Whey/ Casein | 40/60 | 40/60 | 50/50 | 40/60 | 50/50 |
| CHO (g/100 kcal) | 12.9 | 12.9 | 12.3 | 12.9 | 12.3 |
| Lactose (g/100 kcal) | 9 | 12.9 | 7.7 | 12.9 | 7.7 |
| Maltodextrine (g/100 kcal) | 3.9 | | 4.6 | | 4.6 |
| Fat (g/100 kcal) | 4.25 | 4.25 | 4.8 | 4.25 | 4.8 |
| viable bacteria (per g formula dry weight) | (1) 2x10⁸ | (2) Each: 1x10⁸ | (3) Each: 1x10⁸ | (4) 2x10⁸ | (5) 2x10⁸ |
| Prebiotic | (6) | (6) | (6) | (6) | (6) |
| LC-PUFA | | | | DHA | ARA / DHA |
| Energy (Kcal/100mL) | 64.88 | 64.88 | 65 | 64.88 | 65 |

Specifications of the bacteria included are as follows:
(1) P. freudenreichii (not according to the present invention); (2) P. freudenreichii and Bifidobacterium lactis BB12; (3) P. freudenreichii and Lactobacillus rhamonosus GG; (4) Megasphera elsdensii (not according to the invention); (5) Veillonella parvula (not according to the invention); (6) Optionally GOS.

## Claims

1. A dry food composition comprising
(a) viable lactic acid-producing bacteria from one or more live bacteria strains; and
(b) viable lactate utilizing, propionic acid-producing bacteria from one or more live bacteria strains; and
(c) optionally prebiotics;
for use in the treatment of digestive diseases or disorders selected from the group consisting of colics, intestinal discomfort, intestinal pain, visceral sensitivity and intestinal cramp.

2. The food composition according to claim 1, for use in the treatment of infantile colics.

3. The food composition according to claim 1 or 2, wherein said viable lactate utilizing, propionic acid-producing bacteria (b) are selected from Propionibacteria, Veillonella, Selomonas, and Megasphaera.

4. The food composition according to claim 3, wherein said viable lactate utilizing, propionic acid-producing bacteria (b) are selected from the strains consisting of P. freudenreichii, P. acidipropionici, P. jensenii, P. thoenii, P. cyclohexanicum, and P. microaerophilum.

5. The food composition according to any of the preceding claims, wherein said viable lactic acid-producing bacteria (a) are selected from the group of Lactic Acid Bacteria and Bifidobacteria.

6. The food composition according to claim 5, wherein said viable lactic acid-producing bacteria (a) are selected from Bifidobacteria, Lactobacilli, Lactococci, Streptococci, Enterococci, Leuconostoc, and Weissella.

7. The food composition according to any of the preceding claims further comprising additives and / or growth enhancing supplements and / or prebiotics.

8. The food composition according to any of the preceding claims, wherein the prebiotics are selected from the group consisting of FOS and GOS.

9. The food composition according to any of the preceding claims, wherein the amount of said viable bacteria is in the range of 10² to 10¹² CFU per gram or per mL of composition.

10. The food composition according to any of the preceding claims, which is an infant nutritional product, preferably baby milk or baby milk powder.

11. The food composition according to any of the preceding claims, (i) which is designed to be administered to infants or young children starting from the age of 6 months and (ii) provides complete nutrition to the infant or child.

12. A baby formula, comprising the dry food composition according to any of claims 1-9,
for use in the treatment of digestive diseases or disorders selected from the group consisting of colics, intestinal discomfort, intestinal pain, visceral sensitivity and intestinal cramp.

13. The baby formula of claim 12 in the form of a baby milk,
for use in the treatment of digestive diseases or disorders selected from the group consisting of colics, intestinal discomfort, intestinal pain, visceral sensitivity and intestinal cramp.

14. The baby formula of claim 12 in the form of a kit of parts, wherein a first part comprises a dry food composition according to any of claims 1 - 9 and a second part comprises a baby formula, particularly a baby milk, free of viable propionic acid producing bacteria,
for use in the treatment of digestive diseases or disorders selected from the group consisting of colics, intestinal discomfort, intestinal pain, visceral sensitivity and intestinal cramp.

## Patentansprüche

1. Eine Trockennahrungszusammensetzung umfassend
a) lebensfähige, Milchsäure-produzierende Bakterien aus einem oder mehreren lebenden Bakterienstämmen;
und
b) lebensfähige, Laktat-verwendende, Propionsäure-produzierende Bakterien aus einem oder mehreren lebenden Bakterienstämmen;
c) optional Präbiotika;
zur Verwendung bei der Behandlung von Verdauungskrankheiten oder Verdauungsstörungen, ausgewählt aus der Gruppe bestehend aus Koliken, Darmstörungen, Darmschmerzen, viszeraler Empfindlichkeit und Darmkrämpfen.

2. Die Nahrungszusammensetzung nach Anspruch 1 zur Verwendung bei der Behandlung von infantilen Koliken.

3. Die Nahrungszusammensetzung nach Anspruch 1 oder 2, wobei die lebensfähigen, Laktat-verwendenden, Propionsäure-produzierenden Bakterien (b) ausgewählt werden aus: Propionibakterien, Veillonella, Selomonen, und Megasphera.

4. Die Nahrungszusammensetzung nach Anspruch 3, wobei die lebensfähigen, Laktat-verwendenden, Propionsäure-produzierenden Bakterien (b) aus den Stämmen bestehend aus P. freudenreichii, P. acidipropionici, P. jensenii, P. thoenii, P. cyclohexanicum, und P. microaerophilum ausgewählt werden.

5. Die Nahrungszusammensetzung nach einem der vorangehenden Ansprüche, wobei die lebensfähigen, Milchsäure-produzierenden Bakterien (a) aus der Gruppe von Milchsäurebakterien und Bifidobakterien ausgewählt werden.

6. Die Nahrungszusammensetzung nach Anspruch 5, wobei die lebensfähigen, Milchsäure-produzierenden Bakterien (a) aus Milchsäurebakterien, Laktobazillen, Laktokokken, Streptokokken, Enterokokken, Leukonostoc, und Weissella ausgewählt werden.

7. Die Nahrungszusammensetzung nach einem der vorangehenden Ansprüche, weiter umfassend Additive und/oder wachstumsfördernde Zusätze und/oder Präbiotika.

8. Die Nahrungszusammensetzung nach einem der vorangehenden Ansprüche, wobei die Präbiotika aus der Gruppe bestehend aus FOS und GOS ausgewählt werden.

9. Die Nahrungszusammensetzung nach einem der vorangehenden Ansprüche, wobei die Menge der lebensfähigen Bakterien im Bereich von 10² bis 10¹² CFU pro Gramm oder pro mL Zusammensetzung ist.

10. Die Nahrungszusammensetzung nach einem der vorangehenden Ansprüche, welche ein Ernährungsprodukt für Kinder ist, bevorzugt Babymilch oder Babymilchpulver.

11. Die Nahrungszusammensetzung nach einem der vorangehenden Ansprüche, (i) welche ausgestaltet ist, um Kindern oder Kleinkindern ab dem Alter von 6 Monaten verabreicht zu werden und (ii) welche eine komplette Ernährung für das Kind oder Kleinkind bereitstellt.

12. Eine Babynahrung, umfassend die Trockennahrungszusammensetzung nach einem der Ansprüche 1-9,
zur Verwendung bei der Behandlung von Verdauungskrankheiten oder Verdauungsstörungen, ausgewählt aus der Gruppe bestehend aus Koliken, Darmstörungen, Darmschmerzen, viszeraler Empfindlichkeit und Darmkrämpfen.

13. Die Babynahrung nach Anspruch 12 in Form von Babymilch,
zur Verwendung bei der Behandlung von Verdauungskrankheiten oder Verdauungsstörungen, ausgewählt aus der Gruppe bestehend aus Koliken, Darmstörungen, Darmschmerzen, viszeraler Empfindlichkeit und Darmkrämpfen.

14. Die Babynahrung nach Anspruch 12 in Form eines Baukastensystems, wobei ein erster Teil eine Trockennahrungszusammensetzung nach einem der Ansprüche 1-9 umfasst und ein zweiter Teil eine Babynahrung umfasst, insbesondere eine Babymilch, frei von lebensfähigen Propionsäure-produzierenden Bakterien,
zur Verwendung bei der Behandlung von Verdauungskrankheiten oder Verdauungsstörungen, ausgewählt aus der Gruppe bestehend aus Koliken, Darmstörungen, Darmschmerzen, viszeraler Empfindlichkeit und Darmkrämpfen.

## Revendications

1. Une composition alimentaire sèche comprenant
a) des bactéries viables produisant l'acide lactique d'une ou plusieurs souches de bactéries vivantes; et
b) des bactéries viables utilisant du lactate et produisant l'acide propionique d'une ou plusieurs souches de bactéries vivantes; et
c) optionnellement des prébiotiques;
pour l'utilisation dans le traitement de maladies digestives ou des troubles digestifs sélectionnés du groupe consistant de coliques, trouble intestinal, douleur intestinale, sensibilité viscérale et crampes intestinales.

2. La composition alimentaire selon la revendication 1, pour l'utilisation dans le traitement des coliques infantiles.

3. La composition alimentaire selon la revendication 1 ou 2, lesdites bactéries viables utilisant du lactate et produisant l'acide propionique (b) étant sélectionnées de Propionibactéries, Veillonella, Selomones, et Megasphéra.

4. La composition alimentaire selon la revendication 3, lesdites bactéries viables utilisant du lactate et produisant l'acide propionique (b) étant sélectionnées des souches consistant de P. freudenreichii, P. acidipropionici, P. jensenii, P. thoenii, P. cyclohexanicum, et P. microaerophilum.

5. La composition alimentaire selon l'une des revendications précédentes, lesdites bactéries viables produisant l'acide lactique (a) étant sélectionnés du groupe consistant de bactéries lactiques et des bifidobactéries.

6. La composition alimentaire selon la revendication 5, lesdites bactéries viables produisant l'acide lactique (a) étant sélectionnées de bifidobactéries, lactobacilles, lactocoques, streptocoques, entérocoques, leuconostoc, et Weissella.

7. La composition alimentaire selon l'une des revendications précédentes, comprenant en outre des additifs et/ou des suppléments de stimulation de croissance et/ou des prébiotiques.

8. La composition alimentaire selon l'une des revendications précédentes, les prébiotiques étant sélectionnées du groupe consistant de FOS et GOS.

9. La composition alimentaire selon l'une des revendications précédentes, la quantité desdites bactéries viables étant comprise entre 10² et 10¹² CFU par gramme ou par mL de composition.

10. La composition alimentaire selon l'une des revendications précédentes, étant un produit alimentaire pour bébés, préférablement du lait pour bébés ou du lait en poudre pour bébés.

11. La composition alimentaire selon l'une des revendications précédentes, (i) étant conçue pour être administrée à des enfants ou des bébés à partir de l'âge de 6 mois et (ii) assurant une alimentation complète de l'enfant ou du bébé.

12. Un aliment pour bébés comprenant la composition alimentaire sèche selon l'une des revendications 1-9,
pour l'utilisation dans le traitement de maladies digestives ou des troubles digestifs sélectionnés du groupe consistant de coliques, trouble intestinal, douleur intestinale, sensibilité viscérale et crampes intestinales.

13. L'aliment pour bébés selon la revendication 12 en forme d'un lait pour bébés,
pour l'utilisation dans le traitement de maladies digestives ou des troubles digestifs sélectionnés du groupe consistant de coliques, trouble intestinal, douleur intestinale, sensibilité viscérale et crampes intestinales.

14. L'aliment pour bébés selon la revendication 12 en forme d'une trousse de pièces, une première partie comprenant une composition alimentaire sèche selon l'une des revendications 1-9 et une deuxième partie comprenant un aliment pour bébés, particulièrement un lait pour bébés, libre de bactéries produisant l'acide propionique,
pour l'utilisation dans le traitement de maladies digestives ou des troubles digestifs sélectionnés du groupe consistant de coliques, trouble intestinal, douleur intestinale, sensibilité viscérale et crampes intestinales.
